# EUROPEAN PATENT SPECIFICATION

(11) **EP 0 954 305 B1**
(45) Date of publication and mention of the grant of the patent: **07.05.2003**
(21) Application number: 97907822.7
(22) Date of filing: 25.02.1997
(51) Int. Cl.: A61K 31/38, A61K 31/54, A61K 31/41, A61P 27/02

(54) **USE OF CARBONIC ANHYDRASE INHIBITORS FOR TREATING MACULAR EDEMA**
VERWENDUNG VON CARBONANHYDRASE-HEMMERN ZUR BEHANDLUNG VON MAKULÄR-ÖDEMEN
TRAITEMENT CONTRE L'OEDEME MACULAIRE PAR UTILISATION D'INHIBITEURS D'ANHYDRASE CARBONIQUE

(30) Priority: 26.02.1996 US 12250 P; 18.03.1996 GB 9605642
(43) Date of publication of application: 10.11.1999
(73) Proprietor: Advanced Research & Technology Institute, Bloomington, IN 47404 (US)
(72) Inventor: SPONSEL, William, Eric, San Antonio, TX 78256 (US); HARRIS, Alon, Bloomington, IN 47402 (US)
(74) Representative: Kyle, Diana
(86) International application number: US9702874
(87) International publication number: WO97030704

(56) References cited:
- GB-A- 2 223 166
- US-A- 4 797 413
- US-A- 5 153 192
- ARCHIVES OF OPHTHALMOLOGY, vol. 106, no. 9, September 1988, USA, pages 1190-1195, XP000197574 S N. COX ET AL: "Treatment of Chronic Macular Edema with Acetazolamide" cited in the application
- ARCHIVES OF OPHTHALMOLOGY, vol. 108, no. 11, November 1990, USA, pages 1524-1525, XP000197573 M. F. MARMOR: "Hypothesis Concerning Carbonic Anhydrase Treatment of Cystoid Macular Edema: Example with Epiretinal Membrane"
- OPHTHALMIC SURGERY, vol. 25, no. 3, March 1994, USA, pages 166-169, XP000197566 HESAMODIN BORHANI ET AL: "Vitreoretinal Toxicity of Acetazolamide Following Intravitreal Administration in the Rabbit Eye"
- KLINISCHE MONATSBLÄTTER FÜR AUGENHEILKUNDE, vol. 202, January 1993, pages 206-211, XP000197567 H. SCHILLING ET AL: "Therapie zystoider und diffuser Makulaödeme nach Uveitis und Katarakt-Chirurgie mit dem Carboanhydrase-Hemmer Acetazolamid (Diamox)"

## Description

### BACKGROUND OF THE INVENTION

Macular edema is swelling within the retina within the critically important central visual zone at the posterior pole of the eye. An accumulation of fluid within the retina tends to distract the neural elements from one another and from their local blood supply, creating a dormancy of visual function in the area. Usually the process is self-limiting, but permanent visual disability occasionally results. Often times, the swelling may take many months to clear. The precise mechanism by which swelling is triggered is uncertain, but it is probable that certain natural metabolic toxins may play an important role in the disease process. Swelling may also follow the implantation of plastic lenses after cataract surgery, particularly if there is a breech in the lens capsule which segregates the vitreous gel from the fluid filled anterior chamber. Long standing macular edema after cataract surgery is one of the most frustrating situations in all of opthamology, and is remarkably common.

It was first reported by Cox et al, Arch. Ophthalmol., Vol. 106, Sept. 1988, pp. 1190-95, that oral acetazolamide (DIAMOX®) can cause resolution of chronic macular edema of various causes. In this study, 16 of 41 patients showed a reproducible response to the drug with partial or complete resolution of edema and improvement of visual acuity. The therapeutic effect occurred in more than half of the patients with inherited outer retinal disease or uveitis, but in none with primary retinal vascular disorders. Additional studies have corroborated Cox's findings with acetazolamide (Fishman et al., Arch. Ophthalmol. 1989; 107:1445-1452 and Chen et at., Invest. Ophthalmol. Vis. Sci. 1991; 31:1914-1918) and others have utilized the carbonic anhydrase inhibitor methazolamide (Fishman et al., Arch. Ophthalmol. 1993; 111:1640-1646).

Studies of patients who have proven to be responsive to acetazolamide treatment typically show pigment epithelial cell dysfunction. These cells, which line the innermost layer of the choroid, have villi-like projections which interdigitate with the retinal photoreceptors. This flexible but intimate association between pigment epithelial cells and photoreceptors is of critical importance to retinal health. The photoreceptors are highly active metabolically and produce waste products at a great rate. The pigment epithelial villi typically absorb catabolites, regenerate photo pigment and provide nutrients via their closely associated choriocapillaris vascular network. Fluorescein angiography of the pigment epithelium in individuals with macular edema who have shown to be responsive to acetazolamide demonstrate leakage of dye into the photoreceptor area. This leakage is inhibited by treatment with acetazolamide.

There are a number of theories as to how carbonic anhydrase inhibitors (CAIs) might work on macular edema however none have been conclusively detemined. At least 14 different etiologic subtypes of macular edema exist, and it is probable that some will prove to be more responsive to topical CAI treatment than other types. Some of the abovementioned studies lay a basis for this.

Another even more common chronic condition which has typically been presumed to be irreversible is macular degeneration. Macular degeneration is the most common cause of acquired legal blindness. Instead of fluid accumulating in the outer retina, hard accumulations of lipofuscin, a metabolic waste product, tend to accumulate between the photoreceptors and the villi of the retina pigment epithelium. These accumulations gradually enlarge, and in their early pathologic phase create discrete accumulations known as drusen. The lipofuscin is belived to accumulate as a result of a process known as apoptosis, a breaking off of the photoreceptor elements. Shedding of the cellular components of the photoreceptors is constantly occurring in a healthy retina. Good retinal pigment epithelial metabolism generally insures a rapid clearance of such catabolic byproducts of vision. It is interesting to consider that an improved local circulation or a stabilization of membrane pH gradience might retard or prevent the accumulation of lipofuscin. As drusen accumulate in number and begin to coalesce, vast areas of retinal photoreceptors become permanently disengaged from their neighboring retinal pigment epithelial villi. The sections of retina so effected become blind. The greatest propensity among the aging population is for drusen to accumulate in the very central area of vision, the macula. Current therapy lacks any substantive clinical scientific basis with zinc in tablet form as one attempted method of treatment. Thus, a method of treating and/or preventing age-related macular degeneration would be welcomed by the medical community.

### SUMMARY OF THE INVENTION

It has now been found that drugs in the class of carbonic anhydrase inhibitors (CAIs) when administered topically are useful in the treatment and/or preventing of macular edema and Age-Related Macular Degeneration (ARMD). CAIs include such drugs as dorzolamide, acetazolamide, methazolamide and other compounds which are described in U.S. Patent Nos. 5,153,192, 5,300,499, 4,797,413, 4,386,098, 4,416,890 and 4,426,388; and in pending patent application 93/16701.

The compounds disclosed in these applications, if able to be formulated as a topical agent, would be considered to be effective in the treatment discussed herein. Dorzolamide, S,S-5,6-dihydro-4-ethylamino-6-methyl-4H-thieno-[2,3-b]thiopyran-2-sulfonaimide-7,7 dioxide hydrochloride and its trans enantiomer are useful in the treatment of ocular hypertension associated with glaucoma. CAIs manifest their activity by inhibiting the enzyme, carbonic anhydrase, and impeding their contribution to aqueous humour formation made by the carbonic anhydrase pathway. CAIs block or impede this inflow pathway by inhibiting carbonic anhydrase. Dorzolamide, which is also known under its trademark, TRUSOPT®. is the first topically effective CAI for clinical use.

The present invention is directed to use of an ophthalmic composition comprising an effective amount of a topical carbonic anhydrase inhibitor for the manufacture of a medicament for topical application for the treatment or prevention of macular edema or age-related macular degeneration. In particular, it has been found that Dorzolamide can effectively improve the vision of patients suffering from macular edema.

In particular, the method of treating and/or preventing macular edema and macular degeneration comprises ropically administering a compound having the formula: 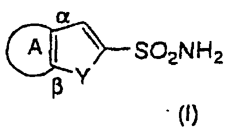 wherein A together with the two carbon atoms denoted as α and β is the group 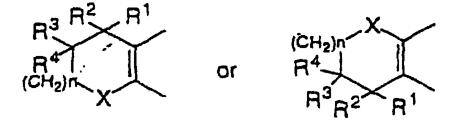 wherein:
- X is: -S-, -SO-, -SO₂- or -CH₂-;
- Y is: -S-, -O-, or -NR³- wherein R³ is hydrogen, C₁₋₃alkyl, or benzyl;
- n is: 1 or 2;
- R¹, R², R³, R⁴ are: independently selected from:
1) hydrogen,
2) OR⁵ wherein R⁵ is:
   a) hydrogen,
   b) C₁₋₅ alkyl, either unsubstituted or substituted with -OH, or 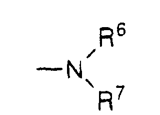 wherein R⁶ and R⁷ are independently hydrogen or C₁₋₅ alkyl, or joined together form a heterocycle with the nitrogen to which they are attached such as piperidino, morpholino, or piperazino,
   c) C₁₋₅ alkanoyl, either unsubstituted or substituted with -OH, -NR⁶R⁷, -NH-COR⁸ or -COR⁸ wherein R⁸ is -OH, -NR⁶R⁷ or C₁₋₅ alkoxy,
   d) -CO-R⁹, wherein R⁹ is -NR⁶R⁷ or a 5- or 6-membered aromatic heterocycle such as pyridyl, imidazolyl, pyrazinyl, thiazolyl, thienyl, or oxazolyl,
3) -NR⁶R⁷,
4) -NHR¹⁰ wherein R¹⁰ is:
   a) -SO₂NR⁶R⁷,
   b) -SO₂R¹¹, wherein R¹¹ is C ₁₋₅ alkyl, or
   c) -CONR⁶R⁷,
5) C₁₋₅ alkyl, either unsubstituted or substituted with
   a) -OR⁵,
   b) -CN,
   c) -NR⁶R⁷, or
   d) -COR⁸,
6) -SO₂R¹¹,
7) -SO₂NR⁶R⁷, or
8) -halo, such as chloro, bromo or fluoro;
- R¹ and R³, or R² and R⁴: taken together represent a double bond;
- R¹ and R², or R³ and R⁴: taken together represent
1) =O, or
2) =NOR¹² wherein R¹² is hydrogen or C₁₋₃alkyl; and one of the -CH₂-groups of -(CH₂)ₙ- can be substituted with -COR⁸, -CH₂R⁸, or -CH₂COR⁸.
The compounds of formula I are described in and prepared by methods set forth in U.S. Patent No. 4,797,413.

Additionally, the method comprises topically administering a thiophene sulfonamide compound having the formula: 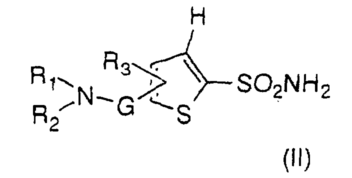 or a pharmaceutically acceptable salt thereof wherein;
- R₁ is: H; C₁₋₄ alkyl; C₂₋₄ alkyl substdtuted optionally with OH, halogen, C₁₋₄ alkoxy or C(=O)R₇.
- R₂ is: H; C₁₋₈ alkyl; C₂₋₈ alkyl substituted with OH, NR₅R₆, halogen, C₁₋₄ alkoxy or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy; C₁₋₃ alkyl substituted with phenyl or heteroaryl which can be unsubstituted or substituted optionally with OH, C(H₂)ₙNR₅R₆, halogen, C₁₋₄ alkoxy, C₁₋₄ haloalkoxy, C(=O)R₇, S(=O)ₘR₈ or SO₂NR₅R₆, wherein m is 0-2 and n is 0-2; C₂₋₄ alkoxy substituted optionally with NR₅R₆, halogen, C₁₋₄ alkoxy, or C(=O)R₇; phenyl, or heteroaryl, unsubstituted or substituted optionally with OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0-2 and n is 0-2; provided that R₁ and R₂ cannot both be H; or R₁ and R2; can be joined to form a saturated ring of 5 or 6 atoms selected from O, S, C or N which can be unsubstituted or substituted optionally on carbon with OH, NR₅R₆, halogen, C₁₋₄ alkoxy, C(=O)R₇, C₁₋₆ alkyl, C₁₋₆ alkyl substituted optionally with OH, NR₅R₆, halogen, C₁₋₄ alkoxy, C(=O)R₇ or on nitrogen with NR₅R₆, C₁₋₄alkoxy, C(=O)R₇, C₁₋₆ alkyl or C₂₋₆ alkyl substituted optionally with OH, NR5R6, halogen, C₁₋₄ alkoxy or C(=O)R₇.
- R₃ is: H; halogen; C₁₋₄ alkyl; C₁₋₈ alkoxy; C₁₋₈ alkylthiol; C₂₋₈ alkoxy substituted optionally with OH, NR₅R₆, halogen, C₁₋₄ alkoxy or C(=O)R₇; C₁₋₄ alkyl substituted optionally with R4; or R₁ and R₃ can be joined together with carbon atoms to form a ring of from 5 to 7 members in wbich said carbon atoms can be unsubstituted or substituted optionally with R₄.
- R₄ is: OH; C₁₋₄ alkyl unsubstituted or substituted optionally with OH, NR₅R₆ halogen, C₁₋₄ alkoxy or C(=O)R₇; C₁₋₄ alkoxy; C₂₋₄ alkoxy substituted optionally with OH, NR₅R₆, halogen, C₁₋₄ alkoxy or C(=O)R₇, NR₅R₆ phenyl, or heteroaryl, unsubstituted or substituted optionally vith OH, (CH₂)ₙNR₅R₆, halogen, C₁₋₄ alkoxy, C₁₋₄ haloalkyoxy, C(=O)R₇, S(=O)ₘR₈ or SO₂NR₅R₆, wherein m is 0-2 and n is 0-2;
provided that when R₃ is in the 4 position and is H or halogen then R₁ and R₂ are not H, C₁₋₆ alkyl substituted optionally with OH, C₁₋₆ alkoxy, C₂₋₆ alkoxycarbonyl, nor are they joined to form a 5, 6 or 7 member ring, saturated or unsaturated, comprised of atoms selected optionally from C, O, S, N in which said nitrogen, when saturated is substituted optionally with H or C₁₋₆ alkyl or in which said carbon is substituted optionally with C₁₋₆ alkyl, C₁₋₆ alkoxy or OH and when R3 is in the 5 position and is H, Cl, Br, or C₁₃ alkyl then neither R₁ nor R₂ can be H or C₁₋₄ alkyl.
- R₅ and R₆ are: the same or different and are H; C₁₋₄ alkyl; C₂₋₄ alkyl substituted optionally with OH, halogen, C₁₋₄ alkoxy or C(=O)R₇; C₁₋₄ alkoxy C₂₋₄ alkoxy substituted optionally with OH, halogen; C₁₋₄ alkoxy or C(=O)R₇; C₃₋₇ alkenyl unsubstituted or substituted optionally with OH; NR₅R₆, or C₁₋₄alkoxy; C₃₋₇alkynyl unsubstituted or substituted optionally with OH, NR₅R₆, or C₁₋₄alkoxy; C₁₋₂alkylC₁₋₃cycloalkyl or R₅ and R₆ can be joined to form a ring of 5 or 6 atoms selected from O, S, C or N which can be unsubstituted or substituted optionally on carbon with OH, (=O), halogen, C₁₋₄; alkoxy, C(=O)R₇, C₁₋₆ alkyl, C₁₋₆ alkyl substituted optionally with OH, halogen, C₁₋₄ alkoxy C(=O)R₇ or on nitrogen with C₁₋₄ alkoxy, C(=O)R₇ S(=O)ₘR₈, C₁₋₆ alkyl or C₂₋₆ alkyl substituted optionally with OH, halogen, C₁₋₄ alkoxy, C(=O)R₇ or on sulfur by (=O)ₘ wherein m is 0-2.
- R7 is: C₁₋₈ alkyl; C₁₋₈ alkyl substituted optionally with OH, NR₅R₆, halogen, C₁₋₄ alkoxy or C(=O)R₉, C₁₋₄alkoxy; C₂₋₄ alkoxy substituted optionally with OH, NR₅R₆, halogen or C₁₋₄ alkoxy; or NR₅R₆.
- R₈ is: C₁₋₄ alkyl; C₂₋₄ alkyl substituted optionally with OH, NR₅R₆, halogen, C₁₋₄ alkoxy or C(=O)R₇.
- R₉ is: C₁₋₄ alkyl; C₁₋₄ alkoxy amino, C₁₋₃ alkylamino, or di-C₁₋₃ alkylamino; and
- G is: C(=O) or SO₂.
The compounds of formula II are described in and prepared by methods set forth in U.S. Patent No. 5,153,192.

Preferred compounds of formula II include compounds of the formula 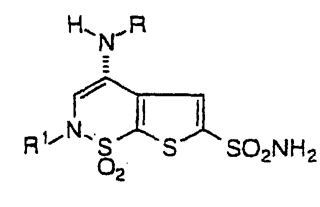 and 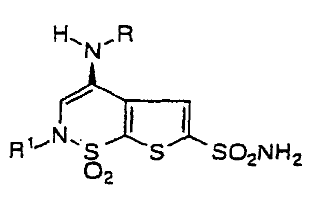 wherein R is selected from -CH₂CH₃ or -CH₂CH₂CH₃ and R¹ is selected from CH₃O(CH₂)₂₋₄ or a pharmaceutically acceptable salt thereof.

The present invention is based upon the discovery that CAIs when administered topically can resolve macular edemas of various causes. It was found that a patient was quickly able to improve visual acuity as measured by an increase in the number of lines the patient was able to read on the standard eye chart after 40 days of topical treatment with a CAI.

Research was done using TRUSOPT®, a particular carbonic anhydrase inhibitor. It is a known compound useful for the reduction of intraocular pressure as described in U.S. Pat. No. 4,797,413.

The CAI used is preferably administered in the form of ophthalmic pharmaceutical compositions adapted for topical administration to the eye such as solutions, ointments or as a solid insert. Formulations of this compound may contain from 0.01 to 5% and especially 0.5 to 2% of medicament. Higher dosages as, for example, about 10% or lower dosages can be employed provided the dose is effective in increasing blood flow velocity. For a single dose, from between 0.001 to 5.0 mg, preferably 0.005 to 2.0 mg, and especially 0.005 to 1.0 mg of the compound is applied to the human eye.

The pharmaceutical preparation which contains the compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, polyalkylene glycols, petroleum based jelly, ethyl cellulose, ethyl oleate. carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, bacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ethylenediamine tetracetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like. The pharmaceutical preparation may also be in the form of a solid insert. For example, one may use a solid water soluble polymer as the carrier for the medicament. The polymer used to form the insert may be any water soluble non-toxic polymer, for example, cellulose derivatives such as methylcellulose, sodium carboxymethyl cellulose, (hydroxyloweralkyl cellulose), hydroxyethyl cellulose, hydroxypropyl cellulose, hydroxypropylmethyl cellulose; acrylates such as polyacrylic acid salts, ethylacrylates, polyactylamides; natural products such as gelatin, alginates, pectins, tragacanth, karaya, chondrus, agar, acacia; the starch derivatives such as starch acetate, hydroxymethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, and mixtures of said polymer.

Preferably the solid insert is prepared from cellulose derivatives such as methylcellulose, hydroxyethyl cellulose, hydroxypropyl cellulose or hydroxypropylmethyl cellulose or from other synthetic materials such as polyvinyl alcohol, polyvinyl pyrrolidone, polyethylene oxide or polyvinyl methylether. Hydroxypropyl cellulose, one of the preferred polymers for the preparation of the insert is available in several polymeric forms, all of which are suitable in the preparation of these inserts. Thus, the product sold by Hercules, Inc. of Wilmington, Delaware under the name KLUCEL™ such as KLUCEL HF, HWF, MF, GF, JF, LF and EF which are intended for food of pharmaceutical use are particularly useful. The molecular weight of these polymers useful for the purposes described herein may be at least 30,000 to about 1,000,000 or more. Similarly, an ethylene oxide polymer having a molecular weight of up to 5,000,000 or greater, and preferably 100,000 to 5,000,000 can be employed. Further, for example, POLYOX™ a polymer supplied by Union Carbide Co. may be used having a molecular weight of about 50,000 to 5,000,000 or more and preferably 3,000,000 to 4,000,000. Other specific polymers which are useful are polyvinyl pyrrolidine having a molecular weight of from about 10,000 to about 1,000,000 or more, preferably up to about 350,000 and especially about 20,000 to 60,000; polyvinyl alcohol having a molecular weight of from about 30,000 to 1,000,000 or more, particularly about 400,000 and especially from about 100,000 to about 200,000; hydroxypropylmethyl cellulose having a molecular weight of from about 10,000 to 1,000,000 or more, particularly up to about 200,000 and especially about 80,000 to about 125,000; methyl cellulose having a molecular weight of from about 10,000 to about 1,000,000 or more, preferably up to about 200,000 and especially about 50 to 100,000; and CARBOPOL™ (carboxyvinyl polymer) of B. F. Goodrich and Co. designated as grades 934,940 and 941.

It is clear that for the purpose of this invention the type and molecular weight of the polymer is not critical. Any water soluble polymers can be used having an average molecular weight which will afford dissolution of the polymer and accordingly the medicament in any desired length of time. The inserts, therefore, can be prepared to allow for retention and accordingly effectiveness in the eye for any desired period. The insert can be in the form of a square, rectangle, oval, circle, doughnut, semi-circle, 1/4 moon shape, and the like. Preferably the insert is in the form of a rod, doughnut, oval or 1/4 moon. The insert can be readily prepared, for example, by dissolving the medicament and the polymer in a suitable solvent and the solution evaporated to afford a thin film of the polymer which can then be subdivided to prepare inserts of appropriate size. Alternatively the insert can be prepared by warming the polymer and the medicament and the resulting mixture molded to form a thin film. Preferably, the inserts are prepared by molding or extrusion procedures well known in the art. The molded or extruded product can then be subdivided to afford inserts of suitable size for administration in the eye. The insert can be of any suitable size to readily fit into the eye. For example, castings or compression molded films having a thickness of about 0.25 mm to 15.0 mm can be subdivided to obtain suitable inserts. Rectangular segments of the cast or compressed film having a thickness between about 0.5 and 1.5 mm can be cut to afford shapes such as rectangular plates of 4x5-20 mm or ovals of comparable size. Similarly, extruded rods having a diameter between about 0.5 and 1.5 mm can be cut into suitable sections to provide the desired amount of polymer. For example, rods of 1.0 to 1.5 mm in diameter and about 20 mm long are found to be satisfactory. The inserts may also be directly formed by injection molding. It is preferred that the ophthalmic inserts containing the medicament of the present invention be formed so that they are smooth and do not have any sharp edges or comers which could cause damage to the eye. Since the term smooth and sharp edges or comers are subjective terms, in this application these terms are used to indicate that excessive irritation of the eye will not result from the use of the insert.

The ocular medicinal inserts can also contain plasticizers, buffering agents and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di and tripropylene glycol, hydroxypropyl sucrose and the like. Typically, such plasticizers can be present in the ophthalmic insert in an amount ranging from up to 1 about 30% by weight. A particularly preferred plasticizer is water which is present in amounts of at least about 5% up to about 40%. In actual practice, a water content of from about 10% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contacting is continued until the water is present in the product in amounts of from about 10% to about 20%.

Suitable water soluble preservatives which may be employed in the insert are sodium bisulfate, sodium thiosulfate, ascorbate, benzalkonium chloride, chlorobutanol, thimerosal, phenylmercuric acetate, phenylmercuric borate, parabens, benzyl alcohol and phenylethanol. These agents may be present in amounts of from about 0.001 to about 5% by weight of solid insert, and preferably about 0.1 to about 2%.

Suitable water soluble buffering agents are alkali, alkali earth carbonates, phosphates, bicarbonates, citrates, borates, and the like, such as sodium phosphate, citrate, borate, acetate, bicarbonate and carbonate. These agents may be present in amounts sufficient to obtain a pH of the system of between about 5.5 to about 8.0 and especially about 7 to about 8; usually up to about 2% by weight of polymer. The insert may contain from about 1 mg to about 100 mg of water soluble polymer, more particularly from about 5 to about 50 mg and especially from about 5 to about 20 mg. The medicament is present from about 0.1 to about 25% by weight of insert.

The claimed use of topical carbonic anhydrase inhibitiors to treat and prevent macular edema has been the subject of a study to determine whether TRUSOPT® drops were effective as a treatment for cystoid macular edema. In the study, a subject with cystoid macular edema in both eyes was administered TRUSOPT® eye drops twice daily as monotherapy. At the pre-treatment fluorescein angiography examination, the patient presented in the early transit a diffuse leakage of dye out of ectatic retinal capillaries widespread over the posterior pole inducing an intensive fuzzy fluorescence. During midtransit, filling of midsized retinal foveal cycts occurred. After 40 days, the patient underwent fluorescein angiography to determine if the therapy had any effect on the edema. The post-treatment fluorescein angiography presented reduced leakage and primarily small and packed aggregate retinal cysts as well as a reading acuity improvement of four lines in the Snellen chart.

## Claims

1. Use of an ophthalmic composition comprising an effective amount of a topical carbonic anhydrase inhibitor for the manufacture of a medicament for topical application for the treatment or prevention of macular edema or age-related macular degeneration.

2. Use according to Claim 1 wherein the carbonic anhydrase inhibitor is a compound of the formula 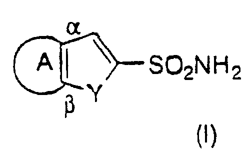 wherein A together with the two carbon atoms denoted as α and β is the group 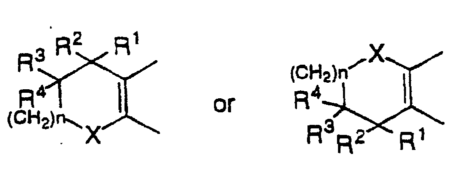 wherein:
X is -S-, -SO-, -SO₂- or -CH₂-;
Y is -S-, -O-, or -NR³- wherein R³ is hydrogen. C₁₋₃alkyl, or benzyl;
n is 1 or 2;
R¹, R², R³, R⁴ are independently selected from:
1) hydrogen,
2) OR⁵ wherein R⁵ is:
a) hydrogen,
b) C₁₋₅ alkyl, either unsubstituted or substituted with -OH, or 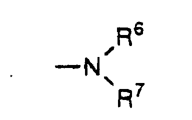
wherein R⁶ and R⁷ are independently hydrogen or C₁₋₅ alkyl, or joined together form a heterocycle

3. Use according to Claim I wherein the carbonic anhydrase inhibitor is a compound of the formula 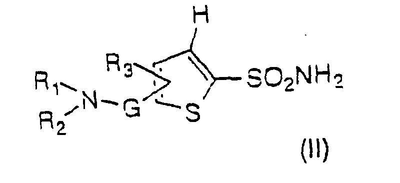 or a pharmaceutically acceptable salt thereof wherein:
R₁ is H; C₁₋₄ alkyl; C₂₋₄ alkyl substituted optionally with OH, halogen. C₁₋₄ alkoxy or C(=O)R₇;
R2 is H; C₁₋₈ alkyl; C₂₋₈ alkyl substituted with OH, NR₅R₆, halogen. C₁₋₄ alkoxy or C(=O)R7; C₃₋₇ alkenyl unsubstituted or substituted optionally with OH, NR₅R₆, or C₁₋₄ alkoxy: C₃₋₇ alkynyl unsubstituted or substituted optionally with OH, NR₅R₆, or C₁₋₄ alkoxy; C₁₋₃ alkyl substituted with phenyl or heteroaryl which can be unsubstituted or substituted optionally with OH, C(H₂)ₙNR₅R₆, halogen, C₁₋₄ alkoxy, C₁₋₄ haloalkoxy, C(=O)R₇, S(=O)ₘR₈ or SO₂NR₅R₆, wherein m is 0-2 and n is 0-2; C₂₋₄ alkoxy substituted optionally with NR₅R₆, halogen, C₁₋₄ alkoxy, or C(=O)R₇; phenyl, or heteroaryl, unsubstituted or substituted optionally with OH, (CH₂)ₙNR₅R₆, halogen. C₁₋₄ alkoxy, C₁₋₄ haloalkoxy, C(=O)R₇, S(=O)ₘR₈ or SO₂NR₅R₆, wherein m is 0-2 and n is 0-2; provided that R₁ and R₂ cannot both be H; or R₁ and R₂, can be joined to form a saturated ring of 5 or 6 atoms selected from O, S, C or N which can be unsubstituted or substituted optionally on carbon with OH, NR₅R₆, halogen, C₁₋₄alkoxy, C(=O)R₇, C₁₋₆ alkyl, C₁₋₆ alkyl substituted optionally with OH, NR₅R₆, halogen, C₁₋₄ alkoxy, C(=O)R₇ or on nitrogen with NR₅R₆, C₁₋₄alkoxy, C(=O)R₇, C₁₋₆ alkyl or C₂₋₆ alkyl substituted optionally with OH, NR₅R₆, halogen. C₁₋₄ alkoxy or C(=O)R₇;
R₃ is H; halogen; C₁₋₄ alkyl; C₁₋₈ alkoxy; C₁₋₈ alkylthiol; C₂₋₈ alkoxy substituted optionally with OH, NR₅R₆, halogen, C₁₋₄ alkoxy or C(=O)R₇; C₁₋₄ alkyl substituted optionally with R₄; or R₁ and R₃ can be joined together with carbon atoms to form a ring of from 5 to 7 members in which said carbon atoms can be unsubstituted or substituted optionally with R₄;

4. Use according to Claim 3 wherein the carbonic anhydrase inhibitor is selected from a compound of the formula 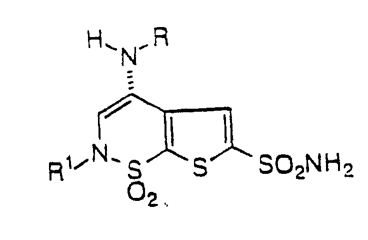 or 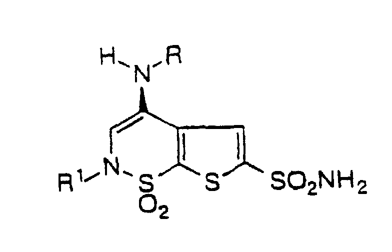 wherein R is selected from -CH₂CH₃ or -CH₂CH₂CH₃ and R¹ is selected from CH₃O(CH₂)₂₋₄ or a pharmaceutically acceptable salt thereof.

5. Use according to Claim 1 wherein the carbonic anhydrase inhibitor comprises dorzolamide, acetazolamide and methazolamide.

6. Use according to Claim 5 wherein the carbonic anhydrase inhibitor is dorzolamide.

7. Use according to claim 1 for the treatment or prevention of age-related macular degeneration.

8. Use according to Claim 7 wherein the carbonic anhydrase inhibitor comprises dorzolamide, acetazolamide and methazolamide.

9. Use according to Claim 7 wherein the carbonic anhydrase inhibitor is dorzolamide.

10. Use according to Claim 7 wherein the carbonic anhydrase inhibitor is a compound of the formula (I) as defined in Claim 2.

11. Use according to Claim 7 wherein the carbonic anhydrase inhibitor is a compound of the formula (II) as defined in Claim 3.

12. Use according to Claim 1 wherein the carbonic anhydrase inhibitor is selected from a compound of the formula 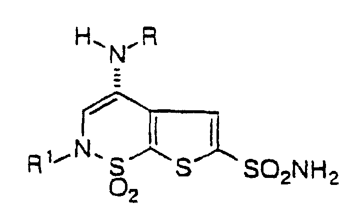 and 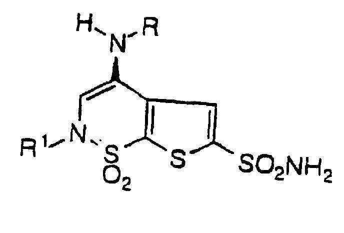 wherein R is selected from -CH₂CH₃ or -CH₂CH₂CH₃ and R¹ is selected from CH₃O(CH₂)₂₋₄ or a pharmaceutically acceptable salt thereof.

## Patentansprüche

1. Verwendung einer ophthalmischen Zusammensetzung, umfassend eine wirksame Menge eines topischen Carbonanhydraseinhibitors für die Herstellung eines Medikamentes zur topischen Anwendung zur Behandlung oder Vorbeugung eines Makulaödems oder einer altersbedingten Makuladegeneration.

2. Verwendung nach Anspruch 1, wobei der Carbonanhydraseinhibitor eine Verbindung der folgenden Formel ist 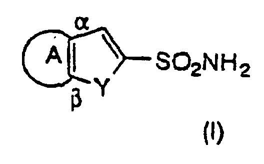 wobei A zusammen mit den beiden Kohlenstoffatomen, die als α und β gekennzeichnet sind, die folgende Gruppe ist 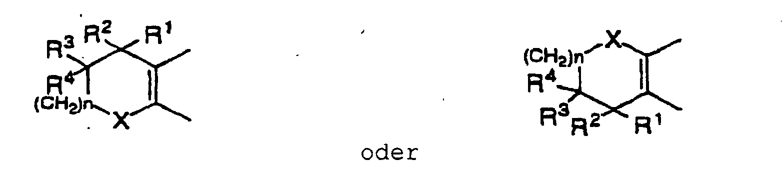 wobei
X -S-, -SO-, -SO₂- oder -CH₂- ist;
Y -S-, -O- oder -NR³- ist, wobei R³ Wasserstoff, C₁₋₃ Alkyl oder Benzyl ist;
n 1 oder 2 ist;
R¹, R², R³, R⁴ unabhängig ausgewählt sind aus:
1) Wasserstoff,
2) OR⁵, wobei R⁵ Folgendes ist:
a) Wasserstoff,
b) C₁₋₅ Alkyl, nichtsubstituiert oder substituiert durch -OH 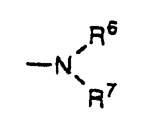 wobei R⁶ und R⁷ unabhängig Wasserstoff oder C₁₋₅ Alkyl oder miteinander verbunden Heterozyklus sind, der mit dem Stickstoff verbunden ist, an das sie angelagert sind, wie Piperidino, Morpholino oder Piperazino;
c) C₁₋₅ Alkanoyl, nichtsubstituiert oder substituiert durch -OH, -NR⁶R⁷, -NH-COR⁸ oder -COR⁸, wobei R⁸ -OH, NR⁶R⁷ oder C₁₋₅ Alkoxy ist;
d) -CO-R⁹, wobei R⁹ -NR⁶R⁷ oder ein 5- oder 6-gliedriger aromatischer Heterozyklus wie Pyridyl, Imidazolyl, Pyrazinyl, Thiazolyl, Thienyl oder Oxazolyl ist;
3) -NR⁶R⁷,
4) -NHR¹⁰, wobei R¹⁰ Folgendes ist:
a) -SO₂NR⁶R⁷,
b) -SO₂R¹¹, wobei R¹¹ C₁₋₅ Alkyl ist, oder
c) -CONR⁶R⁷;
5) C₁₋₅ Alkyl, nichtsubstituiert oder substituiert durch
a) -OR⁵,
b) -CN,
c) -NR⁶R⁷ oder
d) -COR⁸;
6) -SO₂R¹¹
7) -SO₂NR⁶R⁷ oder
8) -Halo, wie Chlor, Brom oder Fluor;
R¹ und R³ oder R² und R⁴ zusammen eine Doppelbindung repräsentieren;
R¹ und R² oder R³ und R⁴ zusammen
1) =O, oder
2) =NOR¹² repräsentieren, wobei R¹² Wasserstoff oder C₁₋₃ Alkyl ist; und eine der -CH₂-Gruppen von -(CH₂)nsubstituiert sein kann durch -COR⁸, -CH₂R⁸ oder -CH₂COR⁸; oder ein pharmazeutisch akzeptables Salz davon.

3. Verwendung nach Anspruch 1, wobei der Carbonanhydraseinhibitor eine Verbindung der folgenden Formel 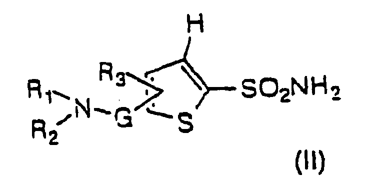 oder ein pharmazeutisch akzeptables Salz davon ist, wobei:
R₁ Folgendes ist: H; C₁₋₄ Alkyl; C₂₋₄ Alkyl, optional substituiert durch OH, Halogen, C₁₋₄ Alkoxy oder C(=O)R₇;
R₂ Folgendes ist: H; C₁₋₈ Alkyl; C₂₋₈ Alkyl, substituiert durch OH, NR₅R₆, Halogen, C₁₋₄ Alkoxy oder C(=O)R7; C₃₋₇ Alkenyl, nichtsubstituiert oder optional substituiert durch OH, NR₅R₆, oder C₁₋₄ Alkoxy; C₃₋₇ Alkynyl, nichtsubstituiert oder optional substituiert durch OH, NR₅R₆, oder C₁₋₄ Alkoxy; C₁₋₃ Alkyl, substituiert durch Phenyl oder Heteroaryl, nichtsubstituiert oder optional substituiert durch OH, C(H₂)ₙNR₅R₆, Halogen, C₁₋₄ Alkoxy, C₁₋₄ Haloalkoxy, C(=O)R₇, S(=O)ₘR₈ oder SO₂NR₅R₆, wobei m 0-2 und n 0-2 ist; C₂₋₄ Alkoxy, optional substituiert durch NR₅R₆, Halogen, C₁₋₄ Alkoxy oder C(=O)R₇; Phenyl oder Heteroaryl, nichtsubstituiert oder optional substituiert durch OH, (CH₂)ₙNR₅R₆, Halogen, C₁₋₄ Alkoxy, C₁₋₄ Haloalkoxy, C(=O)R₇, S(=O)ₘR₈ oder SO₂NR₅R₆, wobei m 0-2 und n 0-2 ist; vorausgesetzt, dass R₁ und R₂ nicht beide H sein können; oder R₁ und R₂ miteinander verbunden werden können, um einen gesättigten Ring mit 5 oder 6 Atomen zu bilden, ausgewählt aus O, S, C oder N, nichtsubstituiert oder optional substituiert auf Kohlenstoff durch OH, NR₅R₆, Halogen, C₁₋₄ Alkoxy, C(=O)R₇, C₁₋₆ Alkyl, C₁₋₆ Alkyl, optional substituiert durch OH, NR₅R₆, Halogen, C₁₋₄ Alkoxy, C(=O)R₇, oder auf Stickstoff durch NR₅R₆, C₁₋₄ Alkoxy, C(=O)R₇, C₁₋₆ Alkyl oder C₂₋₆ Alkyl, optional substituiert durch OH, NR₅R₆, Halogen, C₁₋₄ Alkoxy oder C(=O)R₇;
R₃ Folgendes ist: H; Halogen; C₁₋₄ Alkyl; C₁₋₈ Alkoxy; C₁₋₈ Alkylthiol; C₂₋₈ Alkoxy, optional substituiert durch OH, NR₅R₆, Halogen, C₁₋₄ Alkoxy oder C(=O)R₇; C₁₋₄ Alkyl, optional substituiert durch R₄; oder R₁ und R₃ miteinander verbunden werden können mit Kohlenstoffatomen, um einen Ring aus 5 bis 7 Gliedern zu bilden, wobei die genannten Kohlenstoffatome nichtsubstituiert oder optional substituiert sein können durch R₄;

4. Verwendung nach Anspruch 3, wobei der Carbonanhydraseinhibitor ausgewählt ist aus einer Verbindung der Formel 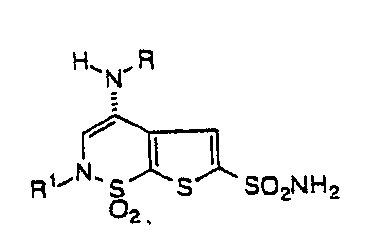 oder 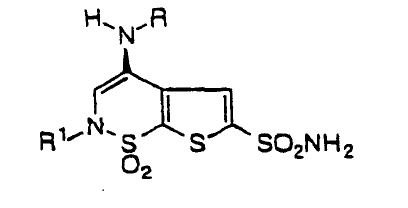 wobei R ausgewählt ist aus -CH₂CH₃ oder -CH₂CH₂CH₃ und R¹ ausgewählt ist aus CH₃O(CH₂)₂₋₄ oder einem pharmazeutisch akzeptablen Salz davon.

5. Verwendung nach Anspruch 1, wobei der Carbonanhydraseinhibitor Dorzolamid, Acetazolamid und Methazolamid umfasst.

6. Verwendung nach Anspruch 5, wobei der Carbonanhydraseinhibitor Dorzolamid ist.

7. Verwendung nach Anspruch 1 zur Behandlung oder Vorbeugung einer altersbedingten Makuladegeneration.

8. Verwendung nach Anspruch 7, wobei der Carbonanhydraseinhibitor Dorzolamid, Acetazolamid und Methazolamid umfasst.

9. Verwendung nach Anspruch 7, wobei der Carbonanhydraseinhibitor Dorzolamid ist.

10. Verwendung nach Anspruch 7, wobei der Carbonanhydraseinhibitor eine Verbindung der Formel (I) gemäß Definition in Anspruch 2 ist.

11. Verwendung nach Anspruch 7, wobei der Carbonanhydraseinhibitor eine Verbindung der Formel (II) gemäß Definition in Anspruch 3 ist.

12. Verwendung nach Anspruch 1, wobei der Carbonanhydraseinhibitor ausgewählt ist aus einer Verbindung der Formel 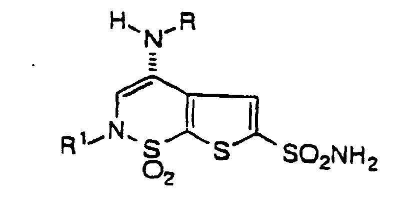 und 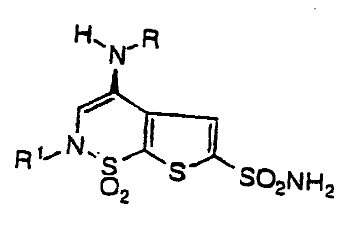 wobei R ausgewählt ist aus -CH₂-CH₃ oder -CH₂CH₂CH₃ und R¹ ausgewählt ist aus CH₃O(CH₂)₂₋₄ oder einem pharmazeutisch akzeptablen Salz davon.

## Revendications

1. Utilisation d'une composition ophtalmique comprenant une quantité efficace d'un inhibiteur topique de l'anhydrase carbonique pour la fabrication d'un médicament d'application topique pour le traitement ou la prévention de l'oedème maculaire ou de la dégénérescence maculaire liée à l'âge.

2. Utilisation selon la revendication 1, dans laquelle l'inhibiteur de l'anhydrase carbonique est un composé de la formule 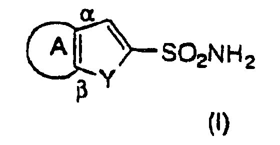 où A, avec les deux atomes de carbone désignés par α et β, est dans le groupe 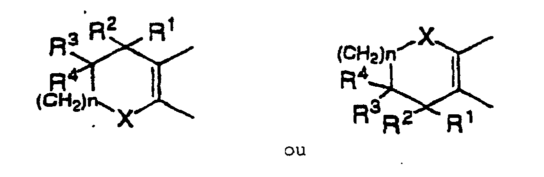 où
X est -S-, -SO-, -SO₂- ou -CH₂- ;
Y est -S-, -O-, ou -NR³-, où R³ est l'hydrogène, le C₁₋₃ alkyle, ou le benzyle ;
n est 1 ou 2 ;
R¹, R², R³, R⁴ sont sélectionnés indépendamment entre :
1) l'hydrogène,
2) OR⁵ où R⁵ est :
a) l'hydrogène,
b) C₁₋₅ alkyle, soit non substitué soit substitué avec -OH 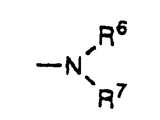 où R⁶ et R⁷ sont indépendamment l'hydrogène ou le C₁₋₅ alkyle, ou un hétérocycle relié avec l'azote auquel ils sont attachés, par exemple piperidino, morpholino ou piperazino
c) C₁₋₅ alkanoyle, soit non substitué, soit substitué avec -OH, -NR⁶R⁷, -NH-COR⁸ ou -COR⁸ où R⁸ est -OH, -NR⁶R⁷, ou C₁₋₅ alkoxy.
d) -CO-R⁹ où R⁹ est -NR⁶R⁷ ou un hétérocycle aromatique à 5 ou 6 membres tel que pyridyle, imidazolyle, pyrazinyle, thiazolyle, thiényle ou oxazolyle.
3) -NR⁶R⁷.
4) -NHR¹⁰ où R¹⁰ est :
a) -SO₂NR⁶R⁷,
b) -SO₂R¹¹, où R¹¹ est C₁₋₅ alkyle, ou
c) -CONR⁶R⁷.
5) C₁₋₅ alkyle, soit non substitué, soit substitué avec
a) -OR⁵,
b) -CN,
c) -NR⁶R⁷, ou
d) -COR⁸,
6) -SO₂R¹¹,
7) -SO₂NR⁶R⁷, ou
8) -halo, tel que chloro, bromo ou fluoro ;
R¹ et R³, ou R² et R⁴ pris ensemble représentent une double liaison ;
R¹ et R², ou R³ et R⁴ pris ensemble
représentent
1) =O, ou
2) =NOR¹² où R¹² est l'hydrogène ou C₁₋₃ alkyle ; et l'un des groupes -CH₂- de -(CH₂)ₙ- peut être substitué avec -COR⁸, -CH₂R⁸, ou -CH₂COR⁸ ;
ou un sel pharmaceutiquement acceptable de ces composés.

3. Utilisation selon la revendication 1, dans laquelle l'inhibiteur de l'anhydrase carbonique est un composé de la formule 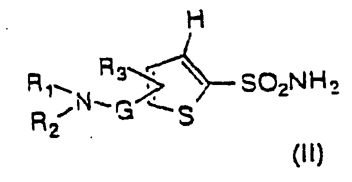 ou un sel pharmaceutiquement acceptable de ce composé, où :
R₁ est H ; C₁₋₄ alkyle ; C₂₋₄ alkyle substitué optionnellement avec OH, halogène, C₁₋₄ alkoxy ou C(=O)R₇ ;
R2 est H ; C₁₋₈ alkyle ; C₂₋₈ alkyle substitué avec OH, NR₅R₆, halogène, C₁₋₄ alkoxy ou C(=O)R7 ; C₃₋₇ alkényle non substitué ou substitué optionnellement avec OH, NR₅R₆, ou C₁₋₄ alkoxy ; C₃₋₇alkynyle non substitué ou substitué optionnellement avec OH, NR₅R₆, ou C₁₋₄ alkoxy ; C₁₋₃ alkyle substitué avec du phényle ou hétéroaryle qui peut être non substitué ou substitué optionnellement avec OH, C(H₂)ₙNR₅R₆, halogène, C₁₋₄ alkoxy, C₁₋₄ haloalkoxy, C(=O)R₇, S(=O)ₘR₈ ou SO₂NR₅R₆, où m est 0-2 et n est 0-2 ; C₂₋₄ alkoxy substitué optionnellement avec NR₅R₆, halogène, C₁₋₄ alkoxy, ou C(=O)R₇, phényle, ou hétéroaryle, non substitué ou substitué optionnellement avec OH, C(H₂)ₙNR₅R₆, halogène, C₁₋₄ alkoxy, C₁₋₄ haloalkoxy, C(=O)R₇, S(=O)ₘR₈ ou SO₂NR₅R₆, où m est 0-2 et n est 0-2 ; étant entendu que R₁ et R₂ ne peuvent pas tous deux être H ; ou R₁ et R₂ peuvent être joints pour former un anneau saturé de 5 ou 6 atomes sélectionnés entre O, S, C ou N qui peuvent être non substitués ou substitués optionnellement sur le carbone avec OH, NR₅R₆, halogène, C₁₋₄ alkoxy, C(=O)R₇, C₁₋₆ alkyle, C₁₋₆ alkyle substitué optionnellement avec OH, NR₅R₆, halogène, C₁₋₄ alkoxy, C(=O)R₇ ou sur l'azote avec NR₅R₆, C₁₋₄ alkoxy, C(=O)R₇, C₁₋₆ alkyle ou C₂₋₆ alkyle substitué optionnellement avec OH, NR₅R₆, halogène, C₁₋₄ alkoxy ou C(=O)R₇ ;
R₃ est H ; halogène, C₁₋₄ alkyle ; C₁₋₈ alkoxy, C₁₋₈ alkylthiol ; C₂₋₈ alkoxy substitué optionnellement avec OH, NR₅R₆, halogène, C₁₋₄ alkoxy ou C(=O)R₇ ; C₁₋₄ alkyle substitué optionnellement avec R₄ ; ou R₁ et R₃ peuvent être reliés ensemble avec des atomes de carbone pour former un anneau de 5 à 7 membres dans lesquels lesdits atomes de carbone peuvent être non substitués ou substitués optionnellement avec R₄ ;

4. Utilisation selon la revendication 3, dans laquelle l'inhibiteur de l'anhydrase carbonique est sélectionné entre un composé de la formule 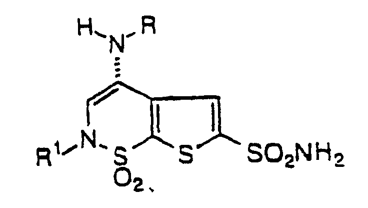 ou 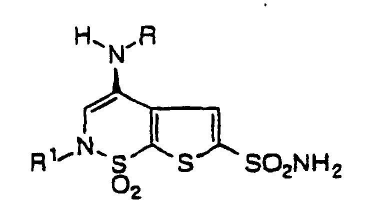 où R est sélectionné entre -CH₂CH₃ ou -CH₂CH₂ CH₃ et R¹ est sélectionné entre CH₃O(CH₂)₂₋₄ ou un sel pharmaceutiquement acceptable de ce composé.

5. Utilisation selon la revendication 1, dans laquelle l'inhibiteur de l'anhydrase carbonique comprend le dorzolamide, l'acétazolamide et le méthazolamide.

6. Utilisation selon la Revendication 5, dans laquelle l'inhibiteur de l'anhydrase carbonique est le dorzolamide.

7. Utilisation selon la revendication 1 pour le traitement ou la prévention de la dégénérescence maculaire liée à l'âge.

8. Utilisation selon la revendication 7, dans laquelle l'inhibiteur de l'anhydrase carbonique comprend le dorzolamide, l'acétazolamide et le méthazolamide.

9. Utilisation selon la revendication 7, dans laquelle l'inhibiteur de l'anhydrase carbonique est le dorzolamide.

10. Utilisation selon la revendication 7, dans laquelle l'inhibiteur de l'anhydrase carbonique est un composé de la formule (I) tel qu'il est défini dans la revendication 2.

11. Utilisation selon la revendication 7, dans laquelle l'inhibiteur de l'anhydrase carbonique est un composé de la formule (II) tel qu'il est défini dans la revendication 3.

12. Utilisation selon la revendication 1, dans laquelle l'inhibiteur de l'anhydrase carbonique est sélectionné entre un composé de la formule 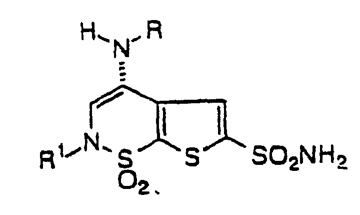 et 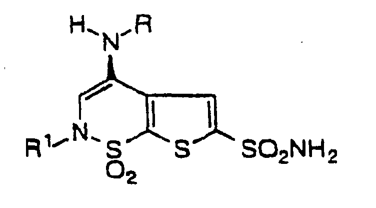 où R est sélectionné entre -CH₂CH₃ ou -CH₂CH₂CH₃ et R¹ est sélectionné entre CH₃0 (CH₂)₂₋₄ ou un sel pharmaceutiquement acceptable de ce composé.
